# NEUE EUROPÄISCHE PATENTSCHRIFT

(11) **EP 0 496 851 B2**
(45) Veröffentlichungstag und Bekanntmachung des Hinweises auf die Entscheidung über den Einspruch: **30.07.1997**
(45) Hinweis auf die Patenterteilung: 26.10.1994
(21) Anmeldenummer: 91913934.5
(22) Anmeldetag: 15.08.1991
(51) Int. Cl.: A61B 17/60

(54) **IMPLANTAT FÜR EINE OSTEOSYNTHESEVORRICHTUNG, INSBESONDERE ZUR WIRBELSÄULENKORREKTUR**
IMPLANT FOR OSTEOSYNTHESIS DEVICE, IN PARTICULAR FOR CORRECTING THE VERTEBRAL COLUMN
IMPLANT POUR DISPOSITIF D'OSTEOSYNTHESE, NOTAMMENT DE CORRECTION DE LA COLONNE VERTEBRALE

(30) Priorität: 21.08.1990 CH 2705/90
(43) Veröffentlichungstag der Anmeldung: 05.08.1992
(73) Patentinhaber: Synthes AG, Chur, CH-7002 Chur (CH)
(72) Erfinder: FRIGG, Robert, CH-7270 Davos-Dorf (CH)
(74) Vertreter: Lusuardi, Werther Giovanni, Dr.
(86) Internationale Anmeldenummer: CH9100174
(87) Internationale Veröffentlichungsnummer: WO9203100

(56) Entgegenhaltungen:
- EP-A- 0 318 356
- EP-A- 0 328 883
- EP-B- 0 348 272
- FR-A- 2 624 720
- FR-A- 2 642 643
- US-A- 2 439 995

## Beschreibung

Die Erfindung bezieht sich auf ein Implantat für eine Osteosynthesevorrichtung, insbesondere zur Wirbelsäulenkorrektur, gemäss der Gattung des Patentanspruchs 1.

Zur Behandlung von Wirbelsäulenverletzungen sind verschiedene Methoden und Osteosynthese-Vorrichtungen bekannt.

Aus der FR-A-2 642 643 ist ein Implantat gemäss der Gattung des Patentanspruch 1 bekannt, bei welchem der koaxiale Zylinder zur Einführung zwischen die Schenkel des oberen Teils des Implantats und der Kappenteil zur Umschliessung der Schenkel aus zwei einzelnen, nicht untereinander verbundenen Teilen besteht. Dies erschwert nicht nur die Handhabung der insgesamt dreiteiligen Pedikelschraube sondern wirkt sich auch negativ auf die Fixation der Schraube am Längsträger aus.
Während der interoperativen Verbindung der meist zahlreichen Pedikelschrauben am Längsträger, hat der koaxiale Zylinder (Madenschraube) die Funktion, den Längsträger in den nach oben offenen Kanal des Schraubenkopfes zu drücken, bevor der Längsträger mit der Pedikelschraube definitiv fixiert wird. Diese Funktion wird praktisch immer benötigt, da die Pedikelschrauben in der Regel nicht immer 90° zur Längsachse des Längsträgers eingebracht werden können. Beim Implantat nach der FR-A-2 642 643 bedeutet dies, daß der separate Kappenteil erst nach Einsetzen der Madenschraube montiert werden kann, was aber durch die inzwischen erfolgte Aufspreizung der beiden Schenkel des Schraubenkopfes (bedingt durch die Einführung der Madenschraube) kaum mehr möglich ist. Anderseits ist ein Aufschieben des Kappenteils vor dem Einbringen der Madenschraube ebenfalls nicht möglich, da der nur teilweise eingesetzte Längsträger (im offenen Kanal des schraubenkopfes) dies verhindert.
Auch die Fixation zwischen Pedikelschraube und Längsträger ist nicht gesichert bei diesem bekannten Implantat. Wenn sich nämlich die Madenschraube durch Bewegungen der Wirbelkörper lockert, kann der Kappenteil sehr leicht vom Schraubenkopf gleiten, so dass die Fixation nicht mehr gewährleistet ist. Der gleiche Nachteil resultiert, wenn die Madenschraube nicht genügend festgezogen wird. In diesem Fall ist der Kappenteil ebenfalls nicht fixiert und kann sehr früh vom Schraubenkopf abgleiten, so dass die ganze Fixation der Wirbelsäule gefährdet ist.

Hier will die Erfindung Abhilfe schaffen. Der Erfindung liegt die Aufgabe zugrunde ein Implantat für eine Osteosynthesevorrichtung, insbesondere für die Wirbelsäulenkorrektur, zu schaffen, welche einfach in der Handhabung, sicher in der Anwendung und platzsparend ist.

Die Erfindung löst die gestellte Aufgabe mit einem Implantat, welches die Merkmale des Anspruchs 1 aufweist.

Das erfindungsgemässe Implantat bewirkt durch die spezielle Konstruktion seines Klemmteils (Kappenteil mit koaxialer, äusserer Krempe zur Umschliessung der Schenkel und koaxialem, gewindetem Innenzylinder zum Einschrauben zwischen die Schenkel aus einem einzigen Stück) eine Selbsthemmung des damit fixierten Längsträgers. In einem ersten Schritt wird durch partielles Festziehen des Innenzylinders der Längsträger provisorisch im offenen Kanal des Schraubenkopfes fixiert. Durch weiteres Festziehen des Klemmteils spreizt sein Innenzylinder die beiden Schenkel geringfügig, wobei diese Spreizung gleichzeitig durch die äussere Krempe des Kappenteils limitiert wird, was zu einer sicheren Verklemmung der Schenkel mit dem Klemmteil führt. Die durch diese Verklemmung entstehende Reibung, verhindert ein ungewolltes Lösen des Klemmteils, durch Bewegungen der Wirbelkörper, da der Innezylinder mit dem Kappenteil fest verbunden ist. Trotz kleinster Dimensionen kann damit das unkontrollierte Spreizen oder Einfallen der Schenkel verhindert werden. Ein unbeabsichtigtes Lösen des Klemmteils und damit des Längsträgers wird damit ausgeschlossen.

Der beim erfindungsgemässen Implantat zentral liegende Klemmmechanismus, gestattet es den Klemmteil dank der Führungsfunktion seines koaxialen, gewindeten Innenzylinders ohne Verklemmungsgefahr in das Gewinde des Implantatkopfes einzuführen.

Insbesondere bei Verwendung des erfindungsgemässen Implantats mit einem gewindeten Längsträger kann der Chirurg die Deformationskorrektur der Wirbelsäule in zwei Schritten durchführen, was zu einer ausserordentlichen Vereinfachung des Eingriffs führt. In einer ersten Phase kann die Korrektur der Wirbelsäule in axialer Richtung durchgeführt werden (Distraktion oder Kompression). Ist dies geschehen, werden die einzelnen Fixationselemente (Pedikelschrauben oder Haken) provisorisch leicht fixiert. Durch dieses leichte Fixieren verkeilt sich das Gewinde des Längsträgers mit dem Kopfteil des Fixationselementes, was zu einer 100%-igen Fixation des erfindungsgemässen Implantats auf dem Längsträger in axialer Richtung führt. In einer zweiten Phase kann nun die Derotation durchgeführt werden. Bei diesem Arbeitsgang verhalten sich die provisorisch fixierten Fixationselemente wie Muttern auf einem Gewindestab, d.h. der Längsträger kann ohne grossen Kraftaufwand und sehr dosiert um seine Längsachse verdreht werden, ohne dass die bereits vorgenommene axiale Korrektur der Wirbelsäule verändert wird. Nach erfolgter Derotation der Wirbelsäule können die einzelnen Fixationselemente definitiv fixiert werden.

Dieser Effekt kann noch zusätzlich verstärkt werden, indem man die Innenpartie des geschlitzten, zur Aufnahme des Längsträgers bestimmten Kopfteils des Implantats ebenfalls mit einem Gewinde versieht.

Anstelle von gewindeten Längsträgern und den damit zur Anlage kommenden gewindeten Innenpartien der Implantat-Kopfteile können auch guergerillte oder andersartig strukturierte Oberflächen verwendet werden.

Bei der bevorzugten Verwendung von strukturierten Oberflächen im erfindungsgemässen Implantat ergeben sich folgende zusätzliche Vorteile:
- Dank der Kombination von gewindeten oder quergerillten Längsträgern, mit gleichartig strukturierten Aufnahmen im Kopf des Implantats, kann eine optimale Verbindung der beiden Elemente erzielt werden;
- die sogenannte Derotation (Rotation des vorgebogenen Langsträgers) kann dank der Oberflächenstruktur der Verbindungselemente, unabhängig von der Distraktion oder Kompression vorgenommen werden;
- die Haltekraft der erzielten Verbindung beschränkt sich nicht auf die Reibhaftung allein, sondern wird durch die Verhakung der Oberflächenstrukturen garantiert.

Bei einer bevorzugten Ausführungsform der Erfindung ist der Innenzylinder des Klemmteils an seinem freien Ende mit einer Spitze versehen um eine eindeutige punktuelle Fixation zu erzielen. Dank der zentrisch angebrachten spitze können auch relativ stark gebogene Längsträger sicher fixiert verden. Anders als bei bekannten Implantatsystemen, welche ebenfalls über eine spitze verfügen, hat die Spitze beim erfindungsgemässen Implantat nur die Funktion den - vorzugsweise strukturierten - Längsträger in die untere Partie des durch die beiden Schenkel gebildeten Kanals zu drücken. Dadurch wird die Pedikelschraube, bzw. der Haken axial am Längsträger fixiert. Die Rotation des Längsträgers im Pedikelschraubenkopf ist demgegenüber von geringerer Bedeutung, da pro Wirbelkörper zwei Pedikelschrauben eingebracht werden und zudem die beiden Längsträger sekundär miteinander verbunden sind.

Bei einer weiteren bevorzugten Ausführungsform bedeckt das Aussengewinde des Innenzylinders des Klemmteils nicht die gesamte Mantelfläche, so dass ein gewindeloser Frontteil resultiert. Die derart gebildete glatte Schulter entspricht im Durchmesser dem Innendurchmesser des Innengewindes des Implantatkopfes. Die enge der Schulter ist so zu wählen, dass sie beim Aufsetzen des Klemmteils, diesen zentriert und in der Gewindelängsachse führt. Ein Verkanten des Klemmteils beim Eindrehen kann dadurch verhindert werden. Diese Führungsfunktion ist bei allen Implantatsystemen mit unterbrochenem Gewinde, d.h. gespaltenem Implantatkopf, wozu auch das erfindungsgemässe Implantat gehört, von grosser Wichtigkeit.

Die Erfindung ist in den Figuren anhand von Ausführungsformen dargestellt, bei welchen der knochenseitige Teil des Implantats aus einer Pedikelschraube besteht. Es sind jedoch auch andere zur Verankerung mit dem Knochen, insbesondere mit den Wirbeln bestimmte Implantatteile für die Erfindung geeignet, beispielsweise in Form von Haken mit einem geschlitzten Kopfteil.
Fig. 1 stellt einen axialen Längsschnitt durch den knochenseitigen Teil des erfindungsgemässen Implantats in Form einer Pedikelschraube dar;
Fig. 2 stellt eine Aufsicht auf den Implantatteil gemäss Fig. 1 mit eingeführtem, aber noch nicht fixiertem Längsträger dar;
Fig. 3 stellt einen axialen Längsschnitt durch den Klemmteil des erfindungsgemässen Implantats dar;
Fig. 4 stellt eine seitliche Ansicht des Klemmteils nach Fig. 3 dar;
Fig. 5 stellt einen Langsschnitt durch das erfindungsgemässe Implantats mit einem darin festgeklemmten Längsträger dar; und

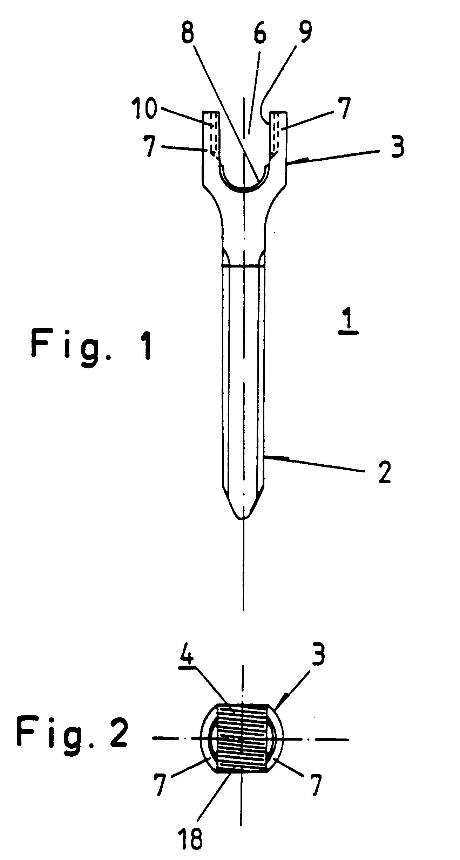

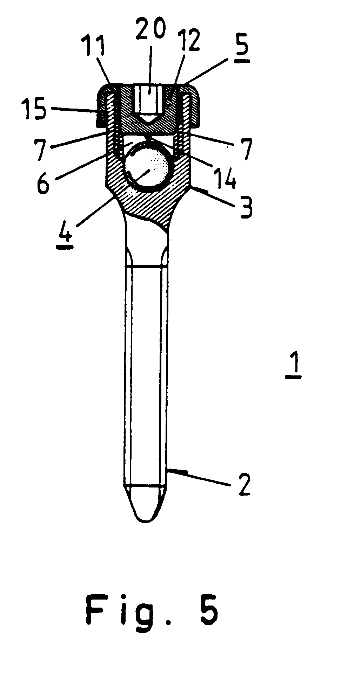

Das in den Fig. 1 bis 4 in seinen Einzelelementen und in Fig. 5 gesamthaft dargestellte Implantat besteht im wesentlichen aus einer Pedikelschraube 1 mit einem unteren, mit einem nicht dargestellten Schraubgewinde versehenen Teil 2 zur Knochenverankerung und einem oberen, als Schraubenkopf ausgebildeten Teil 3 zur lösbaren Fixierung mit einem Langsträger 4 und einem Klemmteil 5.

Der obere Teil 3 weist einen von vorn nach hinten verlaufenden, nach oben offenen Kanal 6 auf, der zwei seitliche Schenkel 7 zur Aufnahme des Längsträgers 4 bestimmt. Die zum unteren Teil 2 gewandte Partie 8 des Kanals 6 ist mit einer Strukturierung versehen, um die Verklemmung mit dem, ein Gewinde 18 aufweisenden Längsträger 4 zu verbessern. Die seitlichen Innenflanken 9 der Schenkel 7 sind mit einem Innengewinde 10 versehen.

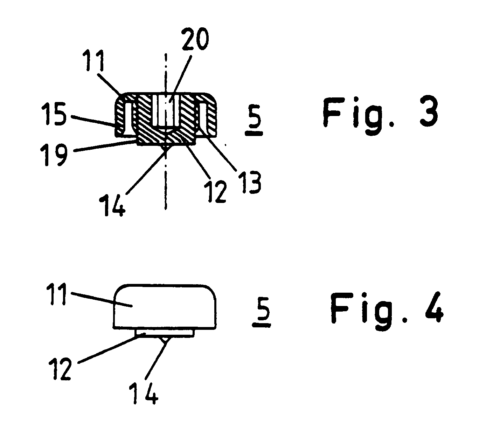

Das in den Fig. 3 und 4 dargestellte Klemmteil 5 besteht im wesentlichen aus einem Kappenteil 11 mit Krempe 15 und einem einstückig damit verbundenen Innenzylinder 12, welcher an seiner äusseren Mantelfläche ein mit dem Innengewinde 10 des Schraubenkopfs 3 korrespondierendes Aussengewinde 13 trägt. An seinem freien Ende ist der Innenzylinder 12 zusätzlich mit einer Spitze 14 versehen. Der Klemmteil 5 weist eine zentrisch im Kappenteil 11 und im Innenzylinder 12 angebrachte Sechskantlochbohrung 20 auf, um das Einschrauben mittels eines geeigneten Instrumentes (Sechskantschlüssel) zu erlauben.

Das Einsetzen des Implantats erfolgt in der Regel so, dass die Pedikelschraube 1 mit ihrem unteren als Schraubgewinde ausgebildeten Teil 2 im Knochen verankert wird und hierauf der Längsträger 4 - wie in Fig. 2 dargestellt - von oben zwischen die beiden ein Innengewinde 10 aufweisende Schenkel 7 des Pedikelschraubenkopfes 3 eingeführt wird.
Hierauf wird das in den Fig. 3 und 4 im Detail dargestellte Klemmteil 5 - wie in Fig. 5 - gezeigt auf den Pedikelschraubenkopf 3 aufgesetzt. Der sich am freien Ende des Innenzylinders 12 befindliche, gewindelose Frontteil 19 weist einen Durchmesser auf, welcher dem Durchmesser des Innengewindes 10 entspricht und eine Länge, welche beim Aufsetzen des Klemmteils 5 dessen sichere Zentrierung garantiert, bis das Aussengewinde 13 des Innenzylinders 12 - in vollständig koaxialer Ausrichtung - mit dem Innengewinde 10 zum Eingriff kommt. Beim Einschrauben des Klemmteils 5 kommt schliesslich die Spitze 14 des Klemmteils 5 auf den Längsträger 4 zu liegen, wodurch dieser sowohl axial als auch rotativ festgeklemmt wird. Gleichzeitig sichert die Krempe 15 des Kappenteils 11 die beiden Schenke 7 des Pedikelschraubenkopfes 3.

## Patentansprüche

1. Implantat für eine Osteosynthesevorrichtung, insbesondere zur Wirbelsäulenkorrektur, mit
A) einem unteren Teil (2) zur Knochenverankerung,
B) einem oberen Teil (3) zur lösbaren Fixierung mit einem Längsträger (4), bei welchem der obere Teil (3) einen von vorn nach hinten verlaufenden, nach oben offenen Kanal (6) aufweist, der zwei seitliche Schenkel (7) zur Aufnahme des Längsträger (4) bestimmt, welche ein Innengewinde (10) aufweisen,
C) einem, zur Einführung zwischen die Schenkel (7) bestimmten, koaxialen Innenzylinder (12), welcher die axiale und rotative Fixierung des Längsträgers (4) gestattet, wobei die äussere Mantelfläche des Innenzylinders (12) ein mit dem Innengewinde (10) des oberen Teils (3) korrespondierendes Aussengewinde (13) aufweist, sowie
D) einem Kappenteil (11) mit koaxialer, äusserer Krempe (15) zur Umschliessung der Schenkel (7),
dadurch gekennzeichnet, dass
der koaxiale Innenzylinder (12) und der Kappenteil (11) als einstückiges Klemmteil (5) für den Längsträger (4) ausgebildet sind.

2. Implantat nach Anspruch 1, dadurch gekennzeichnet, dass die zum unteren Teil (2) gewandte Partie (8) des Kanals (6) mit einer Strukturierung versehen ist.

3. Implantat nach Anspruch 1 oder 2, dadurch gekennzeichnet, dass der Innenzylinder (12) des Klemmteils (5) an seinem freien Ende mit einer, vorzugsweise zentrisch angeordneten Spitze (14) versehen ist.

4. Implantat nach einem der Ansprüche 1 - 3, dadurch gekennzeichnet dass das Aussengewinde (13) des koaxialen Innenzylinder (12) nicht bis zu dessen freiem Ende verläuft, derart dass ein gewindeloser Frontteil (19) des Innenzylinders (12) resultiert.

## Claims

1. Implant for an osteosynthesis device, in particular for spinal column correction, with
A) a lower part (2) to be anchored to the bone,
B) an upper part (3) to be adjustably fixed to a longitudinal bar (4), said upper part (3) being provided with a channel (6) running from front to back and open on the top, which defines two side arms (7) to receive the longitudinal bar (4), whereby said two side arms (7) have an interior thread (10),
C) a coaxial inner cylinder (12) for introduction between the two side arms (7), which allows the axial and rotative fixation of the longitudinal bar (4), whereby the outer surface of the inner cylinder (12) is provided with an exterior thread (13) corresponding to the interior thread (10) of the upper part (3), as well as
D) a cap (11) with a coaxial, outer rim (15) to surround the side arms (7),
**characterized in that**
the coaxial inner cylinder (12) and the cap (11) are designed as a single-piece clamp (5) for the longitudinal bar (4).

2. Implant according to claim 1, characterized in that the portion (8) of the channel (6) facing the lower part (2) is provided with a patterned surface.

3. Implant according to claim 1 or 2, characterized in that the inner cylinder (12) of the clamp (5) is provided with a point (14) at its free end, preferably in the center thereof.

4. Implant according to one of the claims 1 - 3, characterized in that the exterior thread (13) of the coaxial inner cylinder (12) does not run completely to the latter's free end resulting in an unthreaded front portion (19) of the inner cylinder (12).

## Revendications

1. Implant pour dispositif d'ostéosynthèse, en particulier pour la correction de la colonne vertébrale, avec:
A) une partie inférieure (2) destinée à l'ancrage à l'os,
B) une partie supérieure (3) destinée à permettre une fixation démontable à un support longitudinal (4), implant dans lequel la partie supérieure (3) présente un canal (6) ouvert vers le haut, s'étendant de l'avant vers l'arrière, définissant deux branches (7) latérales destinées à recevoir le support longitudinal (4), les deux branches (7), présentant un taraudage (10),
C) un cylindre intérieur (12) coaxial destiné à l'introduction entre les branches (7), et permettant la fixation axiale et rotative du support longitudinal (4), la surface d'enveloppe extérieure du cylindre intérieur (12) présentant un filetage extérieur (13) correspondant au taraudage (10) de la partie supérieure (3) ainsi que,
D) une partie capuchon (11) avec un rebord extérieur (15) destiné à enclore les branches (7),
**caractérisé en ce que** ,
le cylindre intérieur (12) coaxial et la partie capuchon (11) sont réalisés sous forme de parties de serrage (5) monopièce pour le support longitudinal (4).

2. Implant selon la revendication 1, caractérisé en ce que la partie (8), tournée vers la partie inférieure (2), du canal (6) est pourvue d'une structuration.

3. Implant selon la revendication 1 ou 2, caractérisé en ce que le cylindre intérieur (12) de la partie de serrage (5) est pourvue à son extrémité libre d'une pointe (14), de préférence centrée.

4. Implant selon l'une des revendications 1 à 3, caractérisé en ce que le filetage extérieur (13) du cylindre intérieur (12) coaxial ne s'étend pas jusqu'à son extrémité libre, de manière qu'en résulte une partie avant (19) non filetée, du cylindre intérieur (12).
